# EUROPEAN PATENT APPLICATION

(11) **EP 1 902 670 A1**
(43) Date of publication of application: **26.03.2008**
(21) Application number: 06756930.1
(22) Date of filing: 02.06.2006
(51) Int. Cl.: A61B 1/12, A61L 2/24

(54) **MEDICAL INSTRUMENT CLEANING AND DISINFECTING SYSTEM**

(30) Priority: 13.07.2005 JP 2005204755
(71) Applicant: OLYMPUS MEDICAL SYSTEMS CORP., Shibuya-ku Tokyo 151-0072 (JP)
(72) Inventor: NOGUCHI, Toshiaki, OLYMPUS MEDICAL SERVICES CORP., Tokyo 151-0072 (JP); SUZUKI, Eiri, OLYMPUS MEDICAL SYSTEMS CORP., Tokyo 151-0072 (JP)
(74) Representative: Thum, Bernhard
(86) International application number: PCT/JP2006/311109
(87) International publication number: WO 2007/007482

(57) **Abstract**

A medical instrument cleaning/disinfecting system 1 comprises an endoscope 20 in which the opening portions 25g and 25h of a plurality of tube paths 25a and 25b including the aspiration tube path 25c are provided in the proximal portion 21; and the cleaning/disinfecting apparatus 2 provided with the cleaning bath 5 and a plurality of tube path cleaning nozzles 31, 32, and 33; for performing the cleaning/disinfecting process on the external surface of the endoscope 20 after use and the inside of the tube paths 25a, 25b, and 25c. At least the opening portion 25g of the aspiration tube path 25c among a plurality of opening portions 25g and 25h provided in the proximal portion 21 of the endoscope 20 is configured to be spaced by a predetermined distance from the other opening portions 25h of the tube paths 25a and 25b. The cleaning nozzle attaching/detaching mechanism portion 30 is configured to automatically attach and detach a plurality of tube path cleaning nozzles 31, 32, and 33 provided in the cleaning/disinfecting apparatus 2 to and from each of the opening portions 25g and 25h of the proximal portion 21. Accordingly, the medical instrument cleaning/disinfecting system 1 can eliminate the trouble of cleaning and disinfecting the endoscope having a tube path after use and cleaning and disinfecting can be performed hygienically.

## Description

### Technical Field

The present invention relates to a medical instrument cleaning/disinfecting system provided with a medical instrument having a tube path and a cleaning/disinfecting apparatus for cleaning and disinfecting an external surface of the medical instrument and an inside of the tube path.

### Background Art

An endoscope for use in diagnostic examination or treatment of an internal body cavity has a problem in that filth adheres not only to the external surface of an insertion portion to be inserted into the body cavity, but also to the inside of the tube path such as an air supply/water supply tube path and an aspiration tube path serving as a forceps channel. As for the air supply/water supply tube path, filth may adhere to its distal end portion. As for the aspiration tube path, filth may adhere to the entire surface inside the tube path. Accordingly, the external surface of the endoscope and the inside of the tube path after use are cleaned and disinfected.

For example, Japanese Patent Laid-Open No. H07-308289 discloses an endoscope cleaning/disinfecting apparatus having an endoscope mounting rack capable of maintaining an endoscope holding capacity while increasing cleaning and disinfecting effects with a smaller area of a normal contact portion to be contacted to the endoscope. In this apparatus, the endoscope is placed on a reticulately formed rack and the rack is disposed in a cleaning bath for cleaning and disinfecting.

In the endoscope cleaning/disinfecting apparatus, the endoscope which is placed in the cleaning bath undergoes cleaning by cleaning water and disinfecting by being immersed in a disinfectant solution. After cleaning and disinfecting, the endoscope is flushed with clean water. Some of the endoscopes include an aspiration tube path for aspiring a body fluid, filth, and the like, an air supply tube path for supplying air, a water supply tube path for supplying water, and the like.

Accordingly, when an operator cleans and disinfects the endoscope having a tube path, the operator had to connect to each tube path a cleaning tube disposed in the cleaning/disinfecting apparatus for supplying a cleaning solution and a disinfectant solution, as preparatory to the cleaning/disinfecting process.

However, according to the cleaning/disinfecting apparatus disclosed in Japanese Patent Laid-Open No. H07-308289, when an endoscope is cleaned and disinfected, and an endoscope is placed in a rack an operation of connecting the cleaning tube is required. Therefore, the operation of attaching the endoscope to the cleaning/disinfecting apparatus was very troublesome.

The present invention is made in view of the above problem, and an object of the present invention is to provide a medical instrument cleaning/disinfecting system capable of cleaning and disinfecting by simply attaching a medical instrument such as an endoscope having a tube path to a cleaning/disinfecting apparatus.

### Disclosure of Invention

### Means for Solving the Problem

An endoscope cleaning/disinfecting apparatus in accordance with the present invention comprises; a medical instrument provided with a tube path which is provided therein and whose external surface has an opening portion communicatively connected to the tube path; a connection member provided with a connection portion capable of being connected to the medical instrument; and has a communicative connection path communicatively connected to the tube path through the opening portion when the connection portion is connected; a first detection portion for detecting that the medical instrument is placed in a predetermined position of a bath portion with a predetermined depth enough to place the medical instrument; an operation portion for moving the connection member toward the medical instrument on the basis of the detection of the first detection portion; a second detection portion for detecting the connection between the connection member and the medical instrument; and a control portion for supplying a fluid from a fluid supply source to the communicative connection path on the basis of the detection by the second detection portion.

According to the configuration, the operation portion disposed in the cleaning/disinfecting apparatus allows the connection member to automatically move to the opening portion of the medical instrument and after insertion, automatically supply a fluid into the tube path.

### Brief Description of the Drawings

Figs. 1 to 7 relate to an embodiment of the present invention. Fig. 1 is a perspective view explaining the cleaning/disinfecting apparatus with the top cover opened and includes a partial top plan view;
Fig. 2 is a perspective view explaining an example of a configuration of the medical instrument cleaning/disinfecting system;
Fig. 3 a perspective view explaining the cleaning/disinfecting apparatus with the endoscope placed inside the cleaning bath and with the top cover closed;
Fig. 4 is a top plan view showing a configuration of the cleaning nozzle attaching/detaching mechanism portion;
Fig. 5 is a drawing showing a configuration of the cleaning nozzle attaching/detaching mechanism portion viewed from a rear side thereof in a direction opposite from the Fig. 4;
Fig. 6 is a block diagram explaining a configuration of the medical instrument cleaning/disinfecting system;
Fig. 7 is a drawing explaining an example of a configuration of an attachment state detection portion;
Fig. 8 is a drawing showing a configuration in which an ultrasonic proximity sensor serving as an attachment state detection portion is disposed in an aspiration connection opening;
Fig. 9 is a drawing explaining a configuration of the medical instrument cleaning/disinfecting system in which the ultrasonic proximity sensor serving as the attachment state detection portion is disposed in the aspiration connection opening;
Fig. 10 is a drawing explaining a configuration of the medical instrument cleaning/disinfecting system in which the ultrasonic proximity sensor serving as the attachment state detection portion is disposed at the distal end side of a tube path cleaning nozzle; and
Fig. 11 is a drawing explaining another configuration of the cleaning nozzle attaching/detaching mechanism portion of the cleaning/disinfecting apparatus.

### Best Mode for Carrying Out the Invention

Hereinafter, an embodiment of the present invention will be described with reference to the drawings.

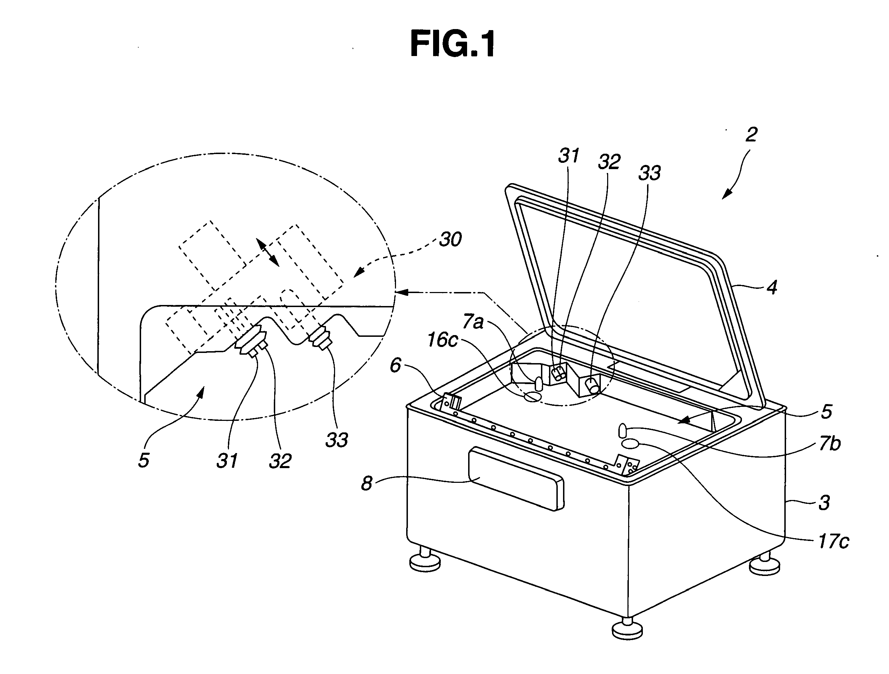

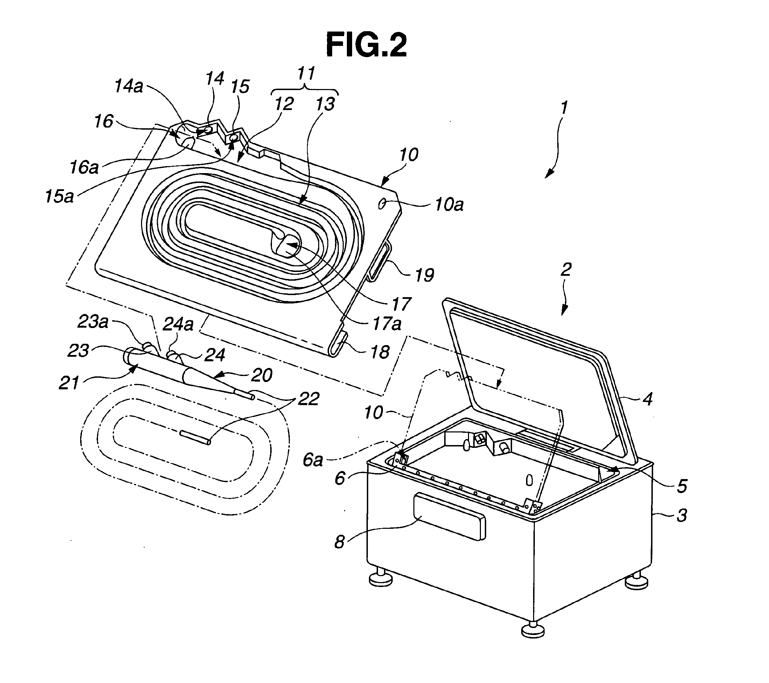

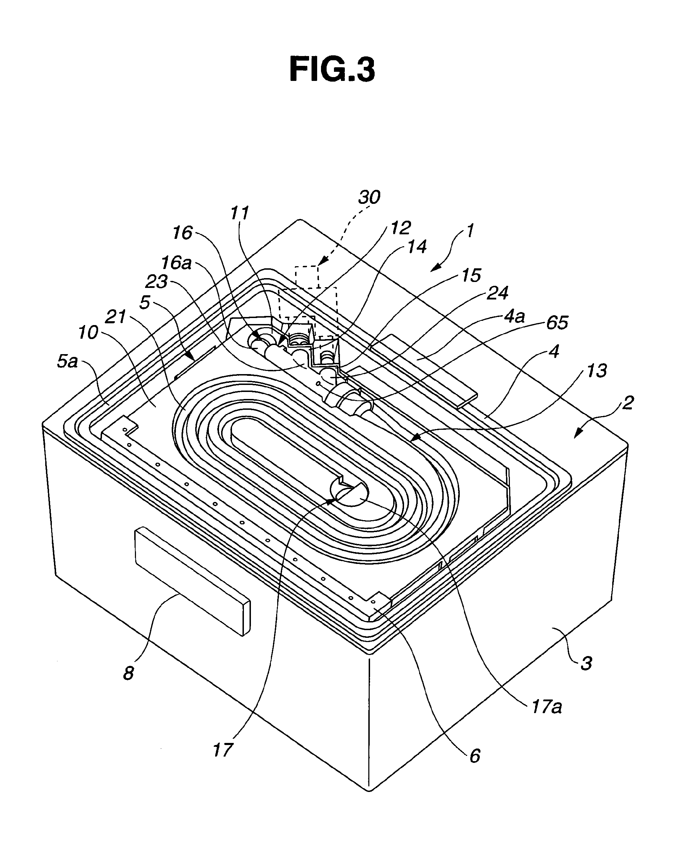

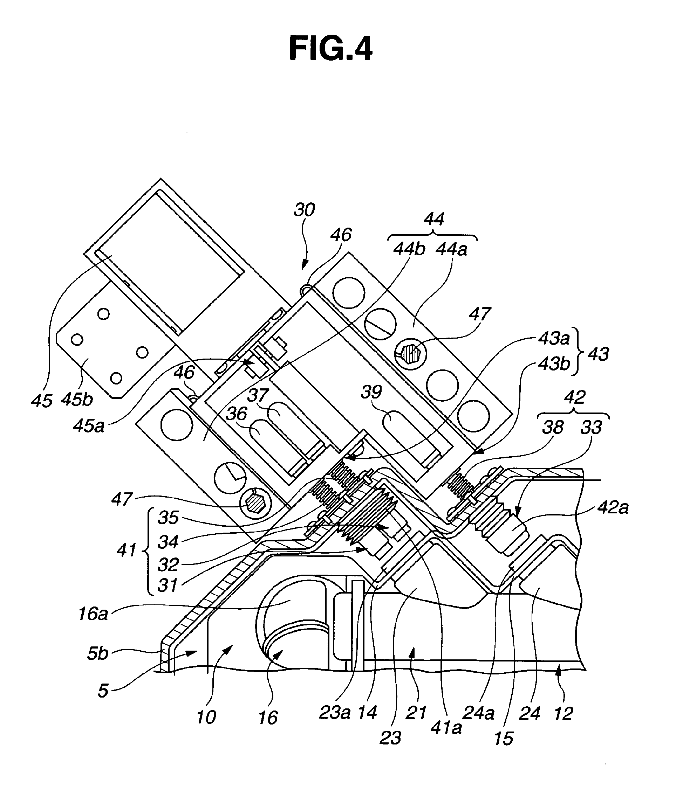

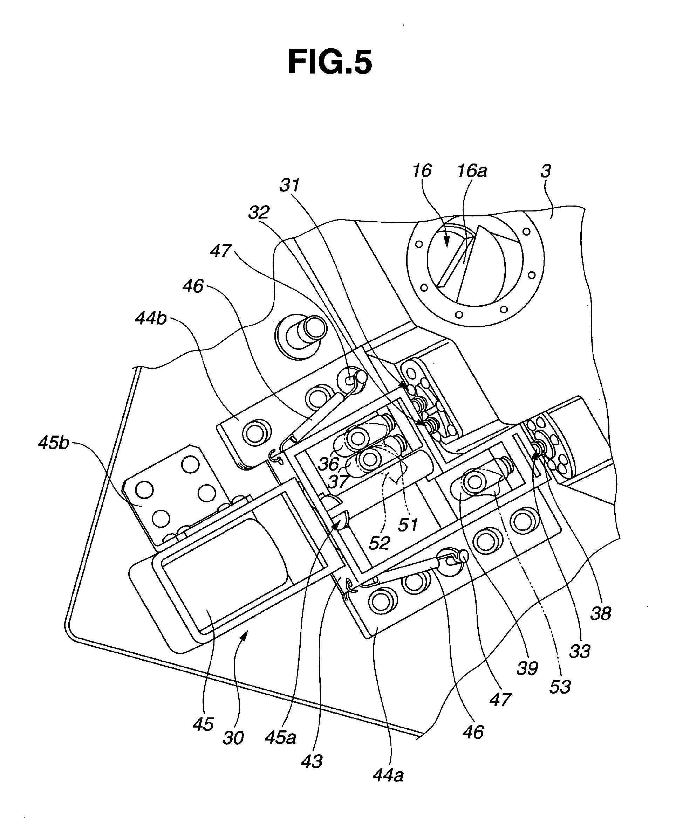

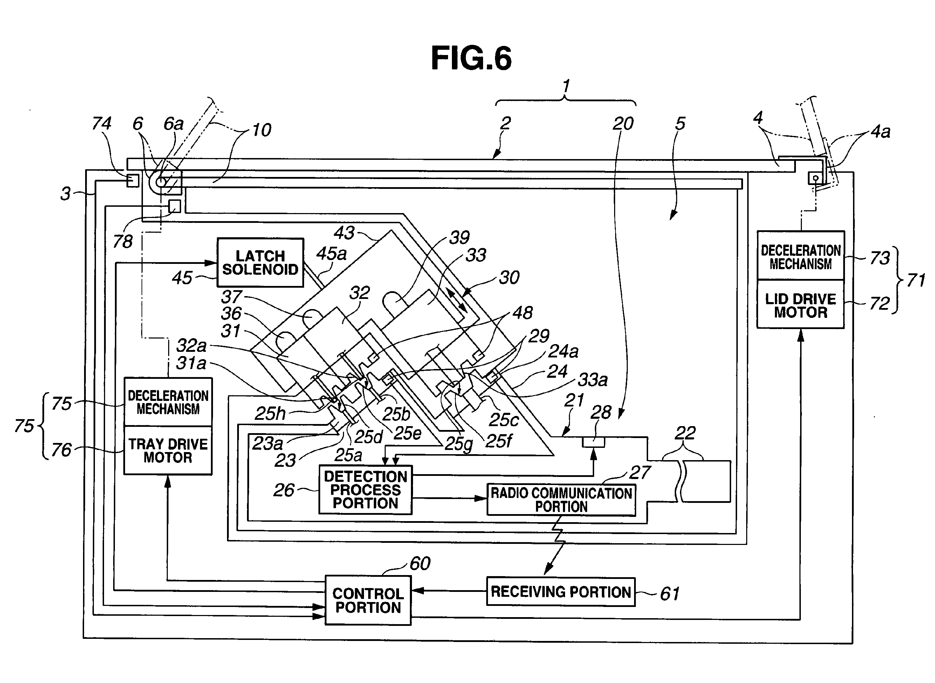

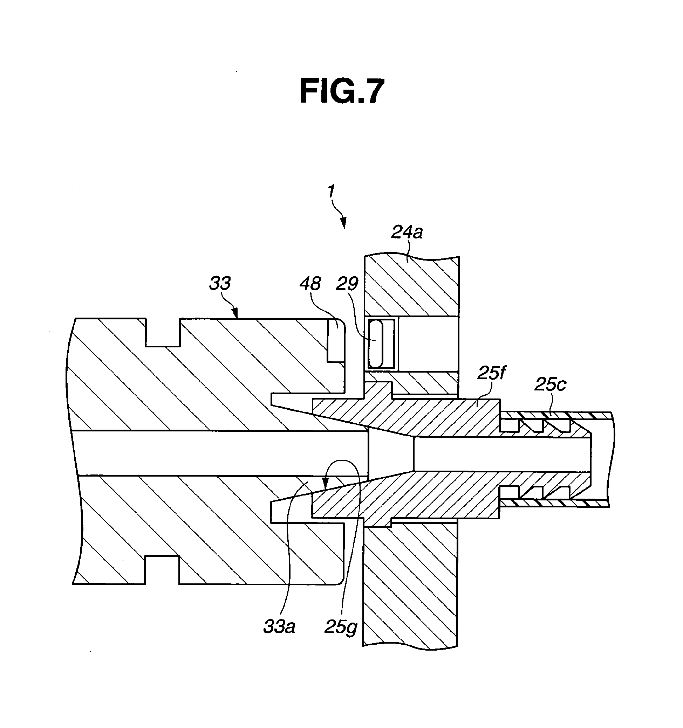

Figs. 1 to 7 relate to an embodiment of the present invention. Fig. 1 is a perspective view explaining the cleaning/disinfecting apparatus with the top cover opened and includes a partial top plan view. Fig. 2 is a perspective view explaining an example of a configuration of the medical instrument cleaning/disinfecting system. Fig. 3 a perspective view explaining the cleaning/disinfecting apparatus with the endoscope placed inside the cleaning bath and with the top cover closed. Fig. 4 is a top plan view showing a configuration of the cleaning nozzle attaching/detaching mechanism portion. Fig. 5 is a drawing showing a configuration of the cleaning nozzle attaching/detaching mechanism portion viewed from a rear side thereof in a direction opposite from the Fig. 4. Fig. 6 is a block diagram explaining a configuration of the medical instrument cleaning/disinfecting system. Fig. 7 is a drawing explaining an example of a configuration of attachment state detection means.

As shown in Fig. 1, the cleaning/disinfecting apparatus 2 constituting the medical instrument cleaning/disinfecting system 1 of the present embodiment comprises an apparatus main body 3 and a top cover 4. A cleaning bath 5 serving as a bath portion with a predetermined depth is disposed on the upper portion of the apparatus main body 3. The top cover 4 is disposed such that the opening of the cleaning bath 5 is covered. A tray holding member 6 is rotatably disposed in a predetermined position of the cleaning bath 5.

A holding tray (shown by the symbol 10 in Fig. 2) is removably disposed on the tray holding member 6. A first opening and closing protrusion 7a and a second opening and closing protrusion 7b are disposed in a predetermined position of the bottom face of the cleaning bath 5. A water inlet opening 16c is disposed in the vicinity of the first opening and closing protrusion 7a and a water outlet opening 17c is disposed in the vicinity of the second opening and closing protrusion 7b. Into the cleaning bath 5 and the holding tray, a cleaning solution and flush water are supplied from the water inlet opening 16c. The cleaning solution and flush water supplied from the water inlet opening 16c are discharged through a water outlet opening 17c out of the cleaning bath 5.

An operation panel 8 allowing various kinds of input operations and capable of displaying characters and the like is disposed in front of the apparatus main body 3.

The top cover 4 is formed into a predetermined shape with a rigid resin member having optical transparency, i.e., a transparent resin member or a translucent resin member. The top cover 4 is disposed in a predetermined position of the cleaning bath 5 such that it can be freely opened and closed. Accordingly, in a state where the opening of the cleaning bath 5 is covered with the top cover 4, visual observation inside the cleaning bath 5 can be made through the top cover 4.

It should be noted that according to the cleaning/disinfecting apparatus 2 of the present embodiment, not only an endoscope (described later), but also a medical instrument such as a treatment instrument and an over tube having an opening portion are cleaned and disinfected. On that occasion, medical instruments are stored in a dedicated holding tray, and the endoscope is stored in an endoscope holding tray (shown by the symbol 10 in Fig. 2) serving as a dedicated holding tray before they are placed in the cleaning bath 5. In addition, the symbols 31, 32, and 33 inside an ellipse shown by a one-dot chain line in Fig. 1 denote tube path cleaning nozzles serving as a connection member for cleaning the inside of a tube path of the endoscope. The tube path cleaning nozzles 31, 32, and 33 constitute a cleaning nozzle attaching/detaching mechanism portion 30 disposed inside the apparatus main body 3 as described later. A tube path cleaning nozzle corresponding to an opening portion disposed in a medical instrument is disposed inside an ellipse shown by a one-dot chain line in Fig. 1.

A medical instrument cleaning/disinfecting system 1 shown in Fig. 2 is an endoscope cleaning/disinfecting system and includes a cleaning/disinfecting apparatus 2, an endoscope holding tray (hereinafter referred to as tray) 10, and an endoscope 20 serving as a medical instrument. The tray 10 is stored and disposed in a holding portion 6a of a tray holding member 6 provided to the cleaning/disinfecting apparatus 2 as shown by a two-dot chain line. The endoscope 20 is stored and disposed in the tray 10.

The endoscope 20 is configured to include a proximal portion 21 and a flexible insertion portion 22 extending from the proximal portion 21. The proximal portion 21 includes an air supply/water supply tube path arrangement portion 23 and an aspiration tube path arrangement portion 24 which are spaced by a predetermined distance and are protruded obliquely in a longitudinal axis direction toward the proximal end side.

An air supply coupling member (see the 25d of Fig. 6) to which an end portion of an air supply tube path (see the symbol 25a of Fig. 6) is coupled and a water supply coupling member (see the symbol 25e Fig. 6) to which an end portion of an air supply tube path (see the symbol 25b of Fig. 6) is coupled are arranged in an air supply/water supply connection opening 23a of the air supply/water supply tube path arrangement portion 23.

On the other hand, only the aspiration tube path (see the symbol 25c of Fig. 6) is arranged in the aspiration tube path arrangement portion 24. An aspiration coupling member (see the symbol 25f of Fig. 6) to which an end portion of the aspiration tube path 25c is coupled is arranged in an aspiration connection opening 24a of the aspiration tube path arrangement portion 24.

The coupling member 25d to be coupled to the air supply tube path 25a, the coupling member 25e to be coupled to the water supply tube path 25b, and the coupling member 25f to be coupled to the aspiration tube path 25c are arranged such that, for example, each central axis thereof is aligned in the same plane.

It should be noted that during endoscopy, an air supply/water supply tube (not shown) for supplying a fluid such as water and air to the endoscope 20 is connected to an air supply/water supply connection opening 23a and an aspiration tube (not shown) to be aspired from the endoscope 20 is connected to aspiration connection opening 24a.

In addition, the proximal portion 21 is used as a grip portion for gripping when the endoscope 20 is operated. In addition, when the endoscope 20 is fixed to an arm and the like, the proximal portion is used as a fixing portion.

A storage concave portion 11 for storing the endoscope 20 in a predetermined position is disposed on the upper surface of the tray 10. The storage concave portion 11 is formed into a predetermined shape by considering the external shape, length, dimension and the like of the proximal portion 21 and the insertion portion 22 of the endoscope 20 to be stored.

In other words, the tray 10 is configured as a dedicated type provided with the storage concave portion 11 for containing a predetermined endoscope 20. Accordingly, in a medical facility using various kinds of endoscopes having a proximal portion 21 and an insertion portion 22 different in external shape, length, and dimension, a plurality of trays corresponding to those various kinds of endoscopes are provided.

More specifically, the storage concave portion 11 includes an operation portion storage portion 12 containing the proximal portion 21 of the endoscope 20 and an insertion portion storage portion 13 containing the insertion portion 22 of the endoscope 20. The operation portion storage portion 12 includes an air supply/water supply tube path arrangement portion receiving portion 14 and an aspiration tube path arrangement portion receiving portion 15 in which the air supply/water supply tube path arrangement portion 23 and the aspiration tube path arrangement portion 24 of the endoscope 20 are arranged respectively.

The air supply/water supply tube path arrangement portion receiving portion 14 includes an air supply/water supply tube path opening 14a in which an air supply/water supply connection opening 23a is installed. On the other hand, aspiration tube path arrangement portion receiving portion 15 includes an aspiration tube path opening 15a in which an aspiration connection opening 24a is installed.

A first water inlet/outlet opening 16 for supplying and discharging cleaning water, disinfectant water, and the like is provided in a predetermined position of the bottom surface of the operation portion storage portion 12. A second water inlet/outlet opening 17 for supplying and discharging cleaning water, disinfectant water, and the like is provided in a predetermined position of the bottom surface of the insertion portion storage portion 13.

The first water inlet/outlet opening 16 is provided in the vicinity of the proximal end side of the proximal portion 21 of the endoscope 20. The second water inlet/outlet opening 17 is provided in the vicinity of the distal end surface side of the insertion portion 22 of the endoscope 20.

Freely opened and closed lid members 16a and 17a are provided in water inlet/outlet openings 16 and 17 respectively. The lid members 16a and 17a are configured to constantly close the water inlet/outlet openings 16 and 17 by their own weights or biasing forces by biasing means (not shown) in addition to the own weights.

In other words, the tray 10 by itself is in a state where the lid members 16a and 17a are closed. Accordingly, in a state where the endoscope 20 after use is stored in the storage concave portion 11 of the tray 10, filth, body fluid, and the like attached to the endoscope 20 can be prevented from leaking through the water inlet/outlet openings 16 and 17. Therefore, the user can hygienically carry the endoscope 20 in a state where the endoscope 20 is stored in the storage concave portion 11 of the tray 10.

According to the present embodiment, a cleaning solution, a disinfectant solution, and the like are fed from the first water inlet/outlet opening 16 to the storage concave portion 11. Then, the cleaning solution, the disinfectant solution, and the like fed into the storage concave portion 11 are discharged from the second water inlet/outlet opening 17 to the cleaning bath 5.

The storage concave portion 11 is formed such that when a cleaning solution, a disinfectant solution, and the like are fed from the first water inlet/outlet opening 16 to the storage concave portion 11, the disinfectant solutions and the like can be sufficiently delivered on the external surface and the like of the proximal portion 21 and the insertion portion 22 of the stored endoscope 20. Accordingly, when the disinfectant solutions and the like are fed to the storage concave portion 11, no wasted space is formed between the endoscope 20 and the storage concave portion 11, thereby allowing a small amount of disinfectant to be used for disinfecting.

It should be noted that the symbol 18 denotes an attaching portion. The attaching portion 18 is provided along one of the longer sides of the tray 10. The attaching portion 18 is formed, for example, in a U-shape to fit in an internal shape of the holding portion 6a constituting the tray holding member 6.

The symbol 19 denotes a transport grasping portion. The transport grasping portion 19 is provided at both the shorter sides of the tray 10 respectively. The transport grasping portion 19 is formed to be projected downward beyond the lower end surface of the tray 10 to prevent interference with the top cover 4.

The symbol 10a denotes a radio tag. The radio tag 10a contains ID information indicating the type and the like of the endoscope to be stored in the storage concave portion 11 of the tray 10.

The user stores the endoscope 20 in the storage concave portion 11 of the tray 10 by inserting the air supply/water supply connection opening 23 a of the air supply/water supply tube path arrangement portion 23 provided in the proximal portion 21 and the aspiration connection opening 24a of the aspiration tube path arrangement portion 24 into the air supply/water supply tube path opening 14a formed at the air supply/water supply tube path arrangement portion receiving portion 14 and the aspiration tube path opening 15a formed at the aspiration tube path arrangement portion receiving portion 15 respectively. By doing this, the user can easily store the endoscope 20 after use in a desired state in the storage concave portion 11 of the tray 10.

The user stores the endoscope 20 in the storage concave portion 11 and then, arranges the tray 10 in the tray holding member 6 as shown by the two-dot chain line in Fig. 2. At this time, when the attaching portion 18 of the tray 10 is arranged in a predetermined state in the holding portion 6a of the tray holding member 6, the tray holding member 6 is moved manually or automatically in a predetermined direction. As the tray holding member 6 is moved, the tray 10 arranged in the tray holding member 6 is stored in a predetermined position inside the cleaning bath 5.

At this time, the first opening and closing protrusion 7a provided on the bottom of the cleaning bath 5 pushes up the lid member 16a to open the first water inlet/outlet opening 16 and at the same time, the second opening and closing protrusion 7b pushes up the lid member 17a to open the second water inlet/outlet opening 17. In addition, as shown in Fig. 4, the air supply/water supply connection opening 23a and the aspiration connection opening 24a of the endoscope 20 arranged to be protruded from the air supply/water supply tube path opening 14a and the aspiration tube path opening 15a of the storage concave portion 11 of the tray 10 respectively are arranged to be spaced by a predetermined distance facing the air supply tube path cleaning nozzle 31, the water supply tube path cleaning nozzle, and the aspiration tube path cleaning nozzle 33 respectively.

Then, the top cover 4 is moved manually or automatically in a predetermined direction to close the opening of the cleaning bath 5 as shown in Fig. 3.

It should be noted that a packing 5a is provided in a predetermined position on the upper face at the opening side of the cleaning bath 5 so as to surround the cleaning bath 5. Therefore, when the opening of the cleaning bath 5 is covered with the top cover 4, the packing 5a is attached firmly to a surface of the top cover 4 to maintain a watertight condition. This can prevent a fluid inside the cleaning bath 5 from splashing outside the apparatus main body 3. The symbol 4a denotes a hinge member which fixes the top cover 4 to cleaning bath 5 such that it can be freely opened and closed.

With reference to Figs. 4 and 5, the configuration of the cleaning nozzle attaching/detaching mechanism portion 30 including tube path cleaning nozzles 31, 32, and 33 will be described.

As shown in the figures, the cleaning nozzle attaching/detaching mechanism portion (hereinafter referred to as mechanism portion) 30 is provided to maintain the attachment state or automatically release the attachment state between the opening portion of the coupling member coupled to a tube path provided in the endoscope 20 stored in the storage concave portion 11 of the tray 10 and the tube path cleaning nozzle provided in the cleaning/disinfecting apparatus 2.

More specifically, in a single operation, the opening portion of the coupling member 25d coupled to the air supply tube path 25a provided in the air supply/water supply connection opening 23a and the opening portion of the coupling member 25e coupled to the water supply tube path 25b are automatically attached to the air supply tube path cleaning nozzle 31 and the water supply tube path cleaning nozzle 32 provided in the cleaning/disinfecting apparatus 2 respectively.

Further, the opening portion of the coupling member 25f coupled to the aspiration tube path 25c provided in the aspiration connection opening 24a are automatically attached to the aspiration tube path cleaning nozzle 33 provided in the cleaning/disinfecting apparatus 2. In addition, the mechanism portion 30 releases the attachment state in a single operation. Therefore, the mechanism portion 30 is provided in a predetermined position with respect to the operation portion storage portion 12 of the tray 10 stored in the cleaning bath 5.

The mechanism portion 30 mainly comprises an air supply/water supply tube path connection portion 41, an aspiration tube path connection portion 42, a nozzle block 43, a pair of rail members 44a and 44b constituting a guide portion 44, a latch solenoid 45 serving as an example of holding means (holding portion) constituting operation means (operation portion) and a pair of connection springs 46 serving as an elastic member constituting biasing means (biasing portion).

The rail members 44a and 44b are arranged parallel in a predetermined position of the apparatus main body 3 to constitute the guide portion 44. The nozzle block 43 is slidably arranged between the guide portion 44 provided with the rail members 44a and 44b.

The nozzle block 43 arranged in the guide portion 44 can be freely moved between the cleaning bath cabinet 5b side and its opposite side. The nozzle block 43 comprises a sliding surface in contact with the rail members 44a and 44b, a solenoid fixing surface to which a solenoid is attached, and a nozzle fixing surface having a shoulder to which the tube path cleaning nozzles 31, 32, and 33 are fixed. The solenoid fixing surface has not only a distal end portion of the solenoid axis 45a of the latch solenoid 45 fixed integrally thereto but also an end portion of each of the connection springs 46 attached thereto.

An attachment plate 45b provided in the latch solenoid 45 is fixed to a predetermined position of the apparatus main body 3. According to the present embodiment, when the cleaning/disinfecting apparatus 2 is in a cleaning/disinfecting wait state, and the solenoid axis 45a is retracted inside the solenoid by the magnetic force inside the solenoid, the latch solenoid 45 is fixed in a position where the nozzle block 43 is in the vicinity of the latch solenoid 45 as shown in Fig. 4.

On the other hand, the other end portion of a pair of connection springs 46 is attached to a pin 47 projected in a predetermined position of the apparatus main body 3. In this spring attached state, the connection springs 46 receive a predetermined biasing force of moving the nozzle block 43 to the cleaning bath cabinet 5b side.

In other words, when the nozzle block 43 is arranged in the vicinity of the latch solenoid 45 as shown in Fig. 4, the magnetic force inside the solenoid is a force by which the nozzle block 43 is holdably arranged in the vicinity of the latch solenoid 45 against the biasing force of the elastically deformed connection springs 46. When the magnetic force inside the solenoid is released, the nozzle block 43 is configured to move to the cleaning bath cabinet 5b side by the biasing force of the connection springs 46.

The air supply/water supply tube path connection portion 41 is configured to include an air supply tube path cleaning nozzle 31, a water supply tube path cleaning nozzle 32, an air supply side buffer spring 34, a water supply side buffer spring 35, an air supply L-shaped pipe 36, and a water supply L-shaped pipe 37. In contrast, the aspiration tube path connection portion 42 is configured to include an aspiration tube path cleaning nozzle 33, an aspiration side buffer spring 38, and an aspiration L-shaped pipe 39.

The tube path cleaning nozzles 31, 32, and 33 are integrally fixed to the nozzle block 43 respectively. At this time, the longitudinal axis of the tube path cleaning nozzles 31, 32, and 33 is, for example, parallel to each other in the same plane.

The air supply tube path cleaning nozzle 31 and the water supply tube path cleaning nozzle 32 are projected by a predetermined amount from the first nozzle fixing surface 43a side constituting the nozzle block 43. Correspondingly, the aspiration tube path cleaning nozzle 33 is also projected by a predetermined amount from the second nozzle fixing surface 43b side constituting the nozzle block 43.

The tube path cleaning nozzles 31, 32, and 33 are inserted into a transparent hole (not shown) provided in the cleaning bath cabinet 5b and its distal end portion is projected into inside the cleaning bath 5. The symbol 41a denotes a watertight holding member configured with an elastic member having a bellows portion and a cover portion for covering the tube path cleaning nozzles 31 and 32. The symbol 42a denotes another watertight holding member configured with an elastic member having a bellows portion and a cover portion for covering the aspiration tube path cleaning nozzle 33.

The tube path cleaning nozzles 31, 32, and 33 positioned between the nozzle block 43 and the cleaning bath cabinet 5b include the air supply side buffer spring 34, the water supply side buffer spring 35, and the aspiration side buffer spring 38 respectively. The buffer springs 34, 35, and 38 serve as a member for buffering a shock occurring when the tube path cleaning nozzles 31, 32, and 33 are inserted into the opening of the coupling member 25d provided inside the air supply/water supply connection opening 23a, the opening of the coupling member 25e, and the opening of the coupling member 25f provided inside the aspiration connection opening 24a.

On the other hand, the L-shaped pipes 36, 37, and 39 are fixed to each of the proximal end portion of the tube path cleaning nozzles 31, 32, and 33 respectively. An air supply tube 51, a water supply tube 52, and an aspiration tube 53 shown by a two-dot chain line are coupled to these L-shaped pipes 36, 37, and 39 respectively. The air supply tube 51, the water supply tube 52, and the aspiration tube 53 are communicatively connected through an electromagnetic valve to an air supply pump, cleaning solution pump, a disinfectant solution pump, and an aspiration pump respectively.

As shown in Figs. 6 and 7, the coupling members 25d and 25e to be coupled to the end portion of the air supply tube path 25a and the end portion of the water supply tube path 25b are provided in the air supply/water supply connection opening 23a of the air supply/water supply tube path arrangement portion 23 of the endoscope 20.

The coupling members 25d and 25e to be coupled to the air supply tube path 25a and the water supply tube path 25b are fixed in a predetermined position of the air supply/water supply connection opening 23a. The other end portions of the air supply tube path 25a and the water supply tube path 25b are coupled to an air supply/water supply nozzle (not shown) provided in the distal end surface of the insertion portion 22.

On the other hand, the coupling member 25f to be coupled to the end portion of the aspiration tube path 25c is provided in the aspiration connection opening 24a of the aspiration tube path arrangement portion 24. The coupling member 25f of the aspiration tube path 25c is fixed in a predetermined position of the aspiration connection opening 24a. The other end portion of the aspiration tube path 25c is communicatively connected to an aspiration opening (not shown) provided on the distal end surface of the insertion portion 22.

In addition, the proximal portion 21 of the endoscope 20 includes a detection process portion 26, a radio communication portion 27 serving as endoscope side radio communication means (endoscope side radio communication portion), a connection complete notification lamp 28, and a reed switch 29. The detection process portion 26 is electrically connected to the radio communication portion 27, the connection complete notification lamp 28, and the reed switch 29. A light emitting portion of the connection complete notification lamp 28 is exposed on the surface of the proximal portion 21 and is placed to be visible externally through the top cover 4.

The reed switch 29 is provided in the air supply/water supply connection opening 23a and the aspiration connection opening 24a respectively. More specifically, the reed switch 29 is placed facing the magnet 48 as shown in Fig. 7.

The magnet 48 is embedded in the distal end surface of the aspiration tube path cleaning nozzle 33 which is attached and detached to and from the aspiration connection opening 24a. In addition, for example, a coupling member 25f having an axial through hole is provided in the aspiration connection opening 24a.

The end portion of the aspiration tube path 25c is fixed to the proximal end portion of the coupling member 25f. The distal end portion of the coupling member 25f includes an opening portion 25g having a tapered surface with its distal opening formed larger in diameter than the through hole.

This opening portion is configured to receive an insertion protrusion 33a constituting the distal end portion of the aspiration tube path cleaning nozzle 33. The distal end surface of the insertion protrusion 33a is projected beyond the distal end surface of the aspiration tube path cleaning nozzle 33 by a predetermined amount.

The tube path cleaning nozzles 31 and 32 also have the insertion protrusions 31a and 32a provided as well respectively. The air supply/water supply connection opening 23a includes the coupling members 25d and 25e having the opening portion 25h with the same configuration as the coupling member 25f and the like. The end portions of the air supply tube path 25a and water supply tube path 25b are fixed to the coupling members 25d and 25e.

As shown in Fig. 7, when the aspiration tube path cleaning nozzle 33 is placed in a predetermined position enough to be inserted into the opening portion 25g of the coupling member 25f provided in the aspiration connection opening 24a, the distance between the reed switch 29 and the magnet 48 is in a predetermined proximity state. Then, the reed switch 29 detects a magnetic force of the magnet 48, and outputs the detection signal to the detection process portion 26.

Meanwhile, when the insertion state is released with the aspiration tube path cleaning nozzle 33 detached from the opening portion 25g of the coupling member 25f, the distance between the reed switch 29 and the magnet 48 gets wider, and the output of a detection signal from the reed switch 29 to the detection process portion 26 is stopped. In other words, the second detection means (the second detection portion) is configured with the magnet 48 and the reed switch 29.

When a detection signal is inputted from the reed switch 29, the detection process portion 26 sends an insertion complete notification signal to the radio communication portion 27 and at the same time outputs a control signal instructing the connection complete notification lamp 28 to light. While the insertion complete notification signal is being inputted from the detection process portion 26, the radio communication portion 27 outputs a cleaning/disinfecting enabled state signal to a receiving portion 61.

Hereinafter, with reference to Fig. 6, the configuration for controlling the mechanism portion 30 will be described.

As shown in the figure, the apparatus main body 3 of the cleaning/disinfecting apparatus 2 includes a control portion 60 serving as control means for performing various kinds of control, and a receiving portion 61. The control portion 60 is electrically connected to the receiving portion 61 and the latch solenoid 45.

The receiving portion 61 receives a cleaning/disinfecting enabled state signal and ID information of the radio tag 10a outputted from the radio communication portion 27 of the endoscope 20. The receiving portion 61 outputs an instruction signal to the control portion 60 in response to the received signal.

More specifically, when a detection signal is outputted from the reed switch 29, and a cleaning/disinfecting enabled state signal is outputted from the radio communication portion 27 to the receiving portion 61, a cleaning/disinfecting process instruction signal instructing the cleaning/disinfecting process to start according to a predetermined procedure is outputted from the receiving portion 61 to the control portion 60. In other words, the cleaning/disinfecting process will not start unless the tube path cleaning nozzles 31, 32, and 33 of the cleaning/disinfecting apparatus 2 are inserted into the opening portions 25h of the coupling members 25d and 25e and the opening portion 25g of the coupling member 25f of the endoscope 20 respectively.

Hereinafter, with reference to Fig. 6, the operation mechanism of the top cover 4 and the tray holding member 6 will be described.

According to the present embodiment, the top cover 4 and the tray holding member 6 are configured to be operable automatically. Therefore, the apparatus main body 3 includes a cover drive portion 71 and a tray drive portion 75. The cover drive portion 71 is configured with a lid drive motor 72 and a deceleration mechanism 73 including a plurality of pulleys and a belt.

On the other hand, the tray drive portion 75 is configured with a tray drive motor 76 and a deceleration mechanism 77. The top cover 4 and the tray holding member 6 are opened or closed accordingly under control of the control portion 60. In addition, the apparatus main body 3 includes a close state stop position sensor 74 for detecting a stop position in which the top cover 4 is closed, and a holding member position detection sensor 78 serving as first detection means (first detection portion) for detecting that the tray 10 is stored in the cleaning bath 5 in a predetermined state. The position detection signals outputted from the sensors 74 and 78 are configured to be sent to the control portion 60 respectively.

In addition, the apparatus main body 3 also includes another sensor (not shown) for detecting that the tray 10 is inserted into the tray holding member 6 and yet other sensors (not shown) for detecting that the top cover 4 and the tray holding member 6 are in an open state respectively.

According to the present embodiment, the tube path cleaning nozzles 31, 32, and 33 are configured, but in addition to the tube path cleaning nozzles, a leak detection nozzle may be configured or one of the tube path cleaning nozzles may be used as the leak detection nozzle. In this configuration, an air for detecting a water leak through the leak detection nozzle is supplied to the endoscope.

Hereinafter the effect of the medical instrument cleaning/disinfecting system 1 will be described.

Before the cleaning/disinfecting apparatus 2 is used, the top cover 4 is in a close state, and the tray 10 is not placed in the tray holding member 6 inside the cleaning bath 5. In addition, a nozzle block 43 of the mechanism portion 30 is moved to the cleaning bath cabinet 5b side by a biasing force of the connection spring 46.

Before cleaning and disinfecting an endoscope, the operator turns on the power of the cleaning/disinfecting apparatus 2 and prepares the tray 10 for storing the endoscope 20 to be cleaned and disinfected. When the power of the cleaning/disinfecting apparatus 2 is turned on, the nozzle block 43 constituting the mechanism portion 30 is moved in the vicinity of the latch solenoid 45 by the magnetic force inside the solenoid.

When the endoscope 20 that has been used for surgery is cleaned, the operator stores the proximal portion 21 and the insertion portion 22 of the endoscope 20 in the storage concave portion 11 of the tray 10. At this time, the operator places the air supply/water supply connection opening 23a and the aspiration connection opening 24a provided in the proximal portion 21 of the endoscope 20, in the air supply/water supply tube path opening 14a of the air supply/water supply tube path arrangement portion receiving portion 14 and the aspiration tube path opening 15a of the aspiration tube path arrangement portion receiving portion 15.

Next, the operator carries the tray 10 storing the endoscope 20 toward the vicinity of the apparatus main body 3. Then, the ID information of the radio tag 10a provided in the tray 10 is transmitted to the control portion 60 through the receiving portion 61 of the apparatus main body 3.

Here, the control portion 60 of the apparatus main body 3 performs a matching with the endoscope data that has been stored in the apparatus main body 3. As a result of the matching, if the control portion 60 determines a match therebetween, an instruction signal is outputted to the cover drive portion 71 and the tray drive portion 75 under control of the control portion 60 to sequentially drive the top cover 4 and tray holding member 6. Then, as shown in Fig. 2, the top cover 4 is opened, and the tray holding member 6 is moved to a position in which the tray 10 can be placed.

Here, the operator places the tray 10 having the endoscope 20 thereon in the holding portion 6a of the tray holding member 6. Then, a sensor (not shown) detects that the tray 10 is placed in the tray holding member 6. Then, the tray holding member 6 and the top cover 4 are closed sequentially under control of the control portion 60.

It should be noted that if the tray 10 cannot be removed from the tray holding member 6, but is configured to be provided integrally to the tray holding member 6, a detection portion may be provided as detection means of detecting that the endoscope is placed in the tray.

When the tray holding member 6 is moved to a closed position, a position detection signal is outputted from the holding member position detection sensor 78 to the control portion 60. At this time, the air supply/water supply connection opening 23a is in a position opposite to the tube path cleaning nozzles 31 and 32, and the aspiration connection opening 24a is in a position opposite to the tube path cleaning nozzles 33 with a predetermined distance therebetween respectively.

Afterward, the top cover 4 is closed and a position detection signal is outputted from the close state stop position sensor 74 to the control portion 60. When the top cover 4 is closed, one of the surfaces of the top cover 4 is attached firmly to the packing 5a to enter a cleaning/disinfecting enabled state. In this state, the endoscope 20 stored in the cleaning bath is visible through the top cover 4.

When a position detection signal is received from aforementioned sensor 78 and another sensor 74, the control portion 60 outputs an insertion instruction signal to the latch solenoid 45 to place the solenoid axis 45a in a release state. Then, the nozzle block 43 is moved to the cleaning bath cabinet 5b side by a biasing force of the connection spring 40.

Then, the tube path cleaning nozzles 31, 32, and 33 integrally fixed to the nozzle block 43 are inserted into the opening portions 25h of the coupling members 25d and 25e provided in the air supply/water supply connection opening 23a, and the aspiration tube path cleaning nozzle 33 is inserted into the opening portion 25g of the coupling members 25f provided in the aspiration connection opening 24a.

When the tube path cleaning nozzles 31 and 32 are inserted firmly into the opening portions 25h of the coupling members 25d and 25e, and the aspiration tube path cleaning nozzle 33 is inserted firmly into the opening portion 25g of the coupling members 25f, the reed switch 29 and the magnet 48 enter a predetermined proximity state and a detection signal is outputted from each of the reed switches 29 to the detection process portion 26.

Then, an insertion complete notification signal is outputted from the detection process portion 26 to the radio communication portion 27 and a control signal is outputted from the detection process portion 26 so as to light the connection complete notification lamp 28. A cleaning/disinfecting enabled state signal is outputted from the radio communication portion 27 to the receiving portion 61.

When the cleaning/disinfecting enabled state signal is received from the radio communication portion 27, the receiving portion 61 outputs the cleaning/disinfecting process instruction signal to the control portion 60. In response to this signal, the cleaning/disinfecting apparatus 2 starts the cleaning/disinfecting process of the endoscope 20 according to a predetermined procedure.

More specifically, according to the cleaning/disinfecting apparatus 2, a disinfectant solution and the like are supplied from the first water inlet/outlet opening 16 and the disinfectant solution and the like are passed through the second water inlet/outlet opening 17 to be discharged to the cleaning bath 5. In addition, at the same time, the disinfectant solution and the like are supplied from the air supply tube path cleaning nozzle 31 to the air supply tube path 25a of the endoscope 20, from the water supply tube path cleaning nozzle 32 to the water supply tube path 25b of the endoscope 20, and from the aspiration tube path cleaning nozzle 33 to the aspiration tube path 25c of the endoscope 20 respectively. The endoscope 20 undergoes a predetermined process such as a cleaning process, a disinfecting process, and a drying process by use of air and then the cleaning/disinfecting process is completed.

After the cleaning/disinfecting process for the endoscope 20 is completed, an insertion state release instruction signal is outputted to the latch solenoid 45 under control of the control portion 60. Then, the solenoid axis 45a is retracted into the solenoid by the magnetic force inside the solenoid.

Then, the nozzle block 43 is moved toward the vicinity of the latch solenoid 45. Then, the tube path cleaning nozzles 31, 32, and 33 integrally fixed to the nozzle block 43 are removed from the opening portions 25h of the coupling members 25d and 25e, and the opening portion 25g of the coupling members 25f provided in the proximal portion 21 of the endoscope 20.

As described above, according to the cleaning/disinfecting apparatus 2 of the present embodiment, an air supply/water supply tube path arrangement portion having an air supply tube path and a water supply tube path provided in the operation portion and an aspiration tube path arrangement portion having only an aspiration tube path are projected obliquely in a longitudinal axis direction toward the proximal end side, and are spaced by a predetermined distance, thereby assuredly preventing filth in the aspiration tube path from spreading into an air supply tube path and a water supply tube path provided inside the air supply/water supply tube path arrangement portion.

In addition, according to the cleaning/disinfecting apparatus 2, an operation portion storage portion is provided in a storage concave portion in which an endoscope is stored. The operation portion storage portion includes an air supply/water supply tube path arrangement portion receiving portion having an air supply/water supply tube path opening and an aspiration tube path arrangement portion receiving portion having an aspiration tube path opening.

When a user stores the endoscope in the tray storage concave portion, the user places the air supply/water supply connection opening of the air supply/water supply tube path arrangement portion in the air supply/water supply tube path opening, and also places the aspiration connection opening of the aspiration tube path arrangement portion in the aspiration tube path opening.

In this state, when the tray is placed in predetermined state in a cleaning bath, the respective tube path cleaning nozzles fixed to a slidable nozzle block constituting a mechanism portion face the opening portions of the air supply/water supply coupling members provided in the air supply/water supply connection opening, and the opening portion of the aspiration coupling member provided in the aspiration connection opening.

When an insertion instruction signal is outputted to a latch solenoid under control of the control portion 60 and the solenoid axis enters a release state, the nozzle block is moved to the cleaning bath cabinet side by the biasing force of a connection spring. Then, each of the tube path cleaning nozzles fixed integrally to the nozzle block is inserted into each of the opening portions of the coupling members provided in the operation portion of the endoscope.

On the other hand, after the cleaning/disinfecting process is completed, an insertion state release instruction signal is outputted to the latch solenoid under control of the control portion 60. Then, the solenoid axis is retracted into the solenoid by the magnetic force inside the solenoid.

Then, the nozzle block is moved to a position opposite to the cleaning bath cabinet. Then, the insertion state between each of the tube path cleaning nozzles fixed integrally to the nozzle block and each of the opening portions of the coupling members is released. In other words, the insertion and removal between a plurality of tube path cleaning nozzles provided in the cleaning/disinfecting apparatus and each of the opening portions of the coupling members provided in the endoscope can be performed automatically.

Accordingly, when an operator performs a cleaning/disinfecting process, the operator can hygienically perform the cleaning/disinfecting process without the need of inserting each of the tube path cleaning nozzles into each of the opening portions of coupling members individually.

In addition, the air supply/water supply tube path arrangement portion and the aspiration tube path arrangement portion provided in the operation portion of the endoscope are spaced by a predetermined distance, and only an aspiration tube path is provided in the aspiration tube path arrangement portion. Accordingly, during the cleaning/disinfecting process, cleaning and disinfectant solutions can be strongly sprayed into the aspiration tube path of the endoscope which is relatively easily contaminated with a body fluid, filth, and the like, thereby maintaining an efficient use of cleaning and disinfectant solutions.

In addition, a plurality of tube path cleaning nozzles are fixed integrally to a nozzle block and this nozzle block is configured to be slidable to and from a latch solenoid by a pair of connection springs.. A plurality of tube path cleaning nozzles can be inserted into or removed from each of the corresponding opening portions of the coupling members by advancing or retreating the nozzle block.

Accordingly, the cleaning/disinfecting apparatus 2 can simplify its configuration and reduce costs as compared to the configuration in which each of the tube path cleaning nozzles is inserted into or removed from the opening portion of the coupling member individually by providing a plurality of latch solenoids.

In addition, according to the cleaning/disinfecting apparatus 2, a magnet is provided in a tube path cleaning nozzle of the cleaning/disinfecting apparatus and a reed switch is provided in a connection opening of the tube path arrangement portion of the endoscope as well. When an insertion protrusion of the tube path cleaning nozzle is inserted into the opening portion of the coupling member in a predetermined state, the reed switch and the magnet are configured to be spaced by a predetermined distance into a predetermined proximity state. Then, a detection signal is outputted from the reed switch.

When a detection signal is outputted from the reed switch, a cleaning/disinfecting enabled state signal is outputted from the radio communication portion to the receiving portion. When the cleaning/disinfecting enabled state signal is received, the control portion 60 starts the cleaning/disinfecting process under control of this control portion 60. This assures that the cleaning/disinfecting process will not start unless the tube path cleaning nozzles are inserted firmly into the opening portions of the coupling members. This can assuredly prevent the cleaning/disinfecting process from starting in an incomplete insertion state. Accordingly, disinfectant solutions and the like can be prevented from being wasted.

According to the present embodiment, the insertion state detection means (insertion state detection portion) is configured with a combination of the reed switch 29 and the magnet 48. However, the insertion state detection means (insertion state detection portion) is not limited to this configuration, but a push switch and a sensor such as a capacitive proximity sensor, an ultrasonic proximity sensor, and a photo interrupter may be used to detect the insertion state in which the nozzle is inserted into the opening portion of the coupling member.

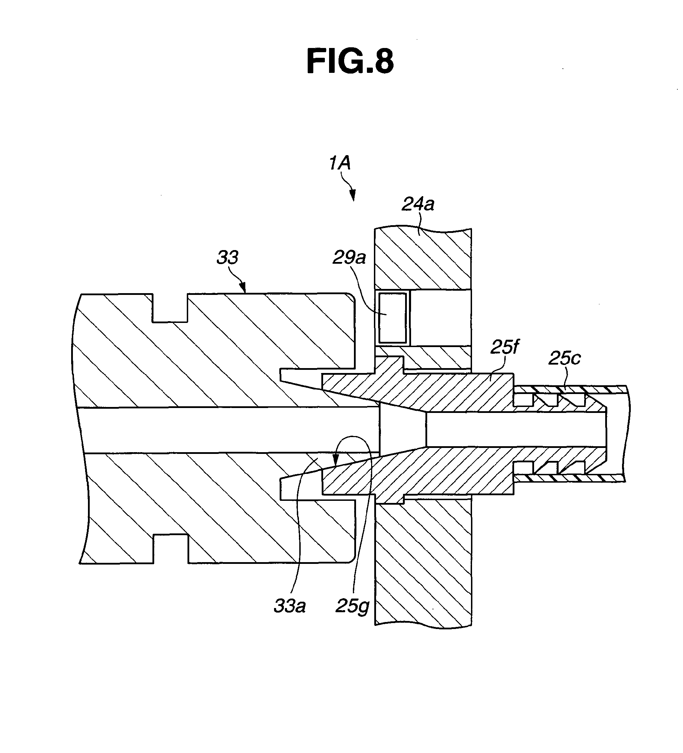

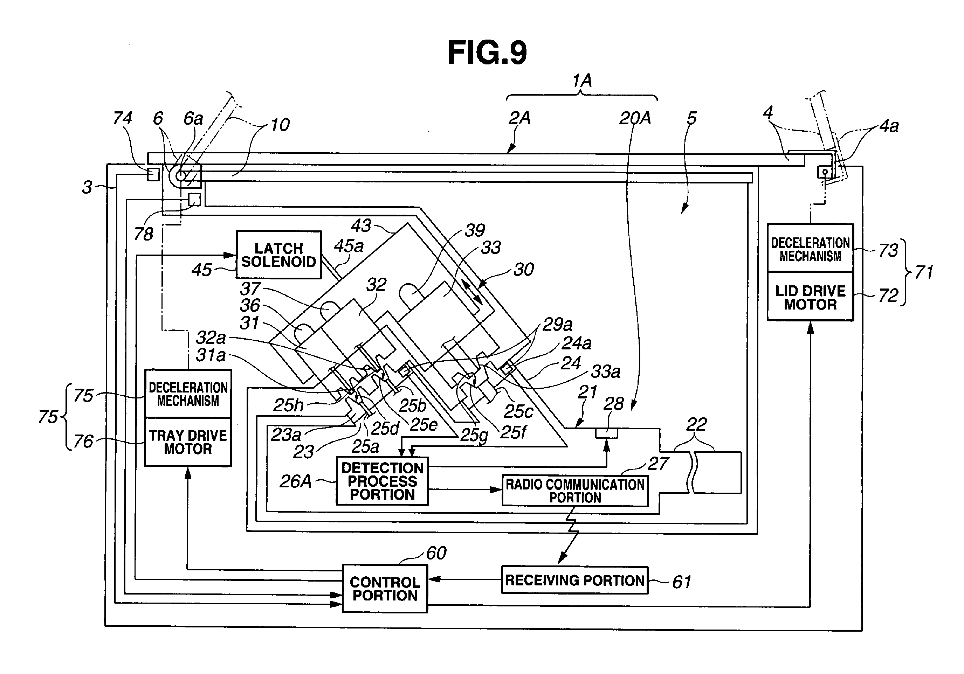

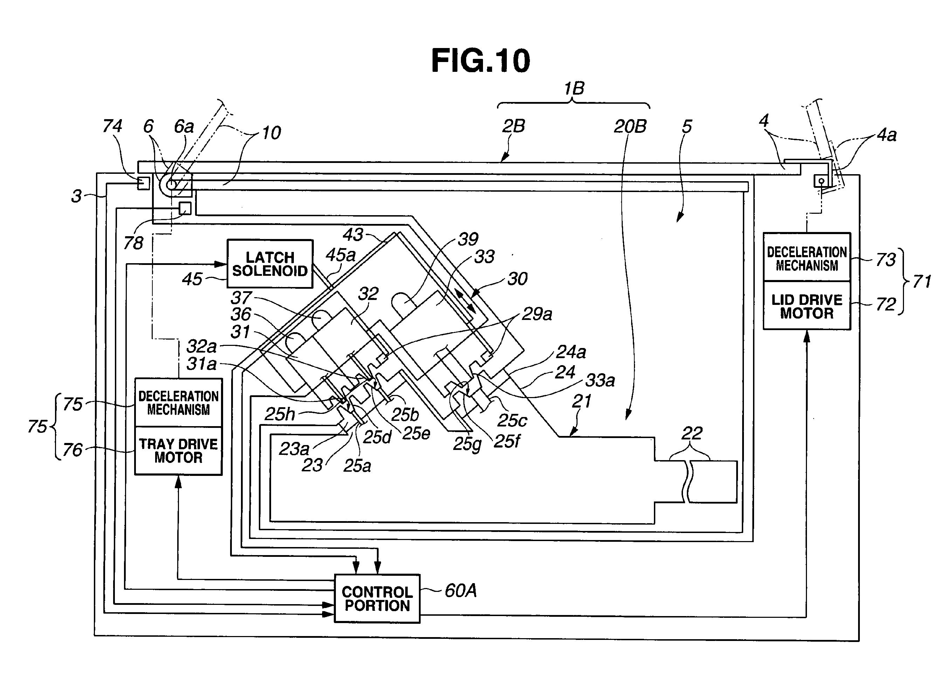

For example, when the ultrasonic proximity sensor is used as the insertion state detection means (insertion state detection portion), a medical instrument cleaning/disinfecting system 1A as shown in Figs. 8 and 9 or a medical instrument cleaning/disinfecting system 1B as shown in Fig. 10 may be configured. Fig. 8 is a drawing showing a configuration in which the ultrasonic proximity sensor serving as the insertion state detection means is provided in the aspiration connection opening. Fig. 9 is a drawing explaining a configuration of the medical instrument cleaning/disinfecting system in which the ultrasonic proximity sensor serving as the insertion state detection means is provided in the aspiration connection opening. Fig. 10 is a drawing explaining a configuration of the medical instrument cleaning/disinfecting system in which the ultrasonic proximity sensor serving as the insertion state detection means is provided at the distal end side of the tube path cleaning nozzle.

As shown in Figs. 8 and 9, according to the medical instrument cleaning/disinfecting system 1A of the present embodiment, the insertion state detection means (insertion state detection portion) does not include the magnet 48 or the reed switch 29, but instead an ultrasonic proximity sensor (hereinafter referred to as ultrasonic sensor) 29a is provided in the air supply/water supply connection opening 23a and the aspiration connection opening 24a of the endoscope 20A. The ultrasonic sensor 29a is electrically connected to the detection process portion 26A.

The ultrasonic sensor 29a detects the distance between the air supply/water supply connection opening 23a and the tube path cleaning nozzles 31 and 32 of the cleaning/disinfecting apparatus 2A as well as the distance between the aspiration connection opening 24a and the aspiration tube path cleaning nozzle 33 of the cleaning/disinfecting apparatus 2A. The detection process portion 26A calculates the distance on the basis of a reflected signal from each of the ultrasonic sensors 29a.

In addition, the detection process portion 26A judges whether or not the calculated distance reaches a predetermined insertion complete distance. If the detection process portion 26A determines that the insertion complete distance is reached, meaning that a completely inserted state is detected, the detection process portion 26A instructs the connection complete notification lamp 28 to light and outputs an insertion complete notification signal to the radio communication portion 27.

Afterward, a cleaning/disinfecting enabled state signal is outputted from the radio communication portion 27 to the receiving portion 61 of the cleaning/disinfecting apparatus 2A. It should be noted that the other configuration, effect, and advantages in the following description are the same as those of the aforementioned embodiment, and thus the same symbols are assigned to the same members and their description is omitted.

As shown in Fig. 10, according to the medical instrument cleaning/disinfecting system 1B, the insertion state detection means (insertion state detection portion) does not include the magnet 48 or the reed switch 29, but instead, for example, an ultrasonic sensor 29a is provided in each of the tube path cleaning nozzles 32 and 33 of the cleaning/disinfecting apparatus 2B. The ultrasonic sensors 29a are electrically connected to the control portion 60A.

The ultrasonic sensor 29a detects the distance between the water supply tube path cleaning nozzle 32 and the air supply/water supply connection opening 23a of the endoscope 20B as well as the distance between the aspiration tube path cleaning nozzle 33 and the aspiration connection opening 24a of the endoscope 20B. The control portion 60A calculates the distance therebetween on the basis of a reflected signal from each of the ultrasonic sensors 29a.

In addition, the control portion 60A judges whether or not the calculated distance reaches a predetermined insertion complete distance. If the control portion 60A determines that the insertion complete distance is reached, meaning that a completely inserted state is detected, the control portion 60A instructs to display characters and the like indicating a connection complete notification on the display portion of the operation panel 8 and then the cleaning/disinfecting apparatus 2 starts the cleaning/disinfecting process of the endoscope 20B under control of the control portion 60A according to a predetermined procedure.

It should be noted that according to the endoscope 20B of the present embodiment, the ultrasonic sensor 29a is provided in the cleaning/disinfecting apparatus 2B. Accordingly, unlike the aforementioned endoscope 20, the endoscope 20B does not have the ultrasonic sensor 29a, the radio communication portion 27, the connection complete notification lamp 28, or the detection process portion 26.

This configuration can simplify the configuration of the endoscope 20B. It should be noted that the other configuration, effect, and advantages in the following description are the same as those of the aforementioned embodiment, and thus the same symbols are assigned to the same members and their description is omitted.

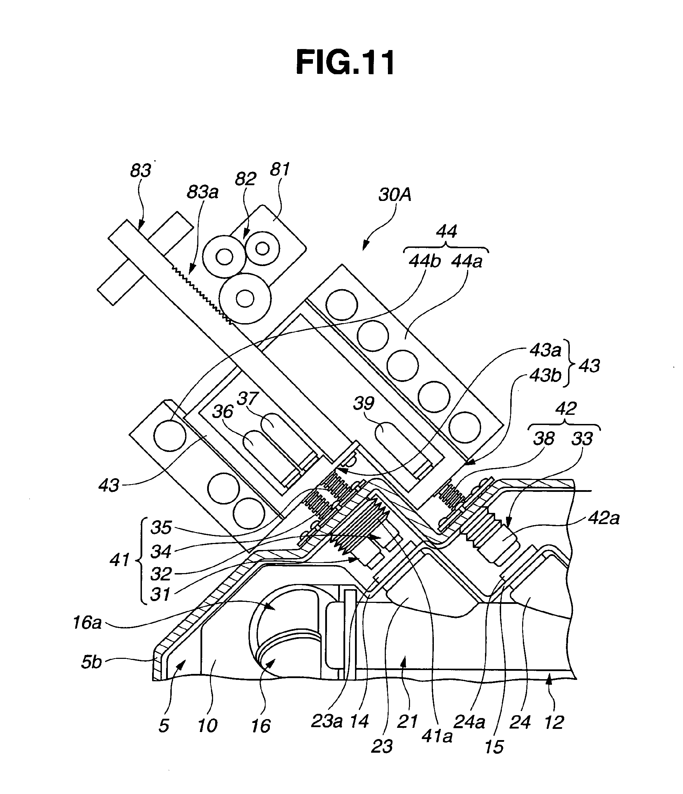

As shown in Fig. 11, a cleaning/disinfecting apparatus of the present embodiment differs only in the configuration of the mechanism portion 30A. Fig. 11 is a drawing explaining another configuration of the cleaning nozzle attaching/detaching mechanism portion of the cleaning/disinfecting apparatus.

The operation means (operation portion) of the mechanism portion 30A provided in the cleaning/disinfecting apparatus 2C does not include the latch solenoid 45 or the connection springs 46, but instead includes advance/retreat means (advance/retreat portion) having a removable drive motor 81 as described later. The advance/retreat means (advance/retreat portion) is configured to transmit the drive power of the removable drive motor 81 to the mesh portion 83a provided in the drive rack axis 83 through the deceleration mechanism 82 having a plurality of gears.

The removable drive motor 81 is fixed in a predetermined position and the drive rack axis 83 advances or retreats with the rotation of the removable drive motor 81. In other words, by the deceleration mechanism 82 that is transmission means (transmission portion) and the mesh portion 83a, the rotational motion of the removable drive motor 81 that is drive means (drive portion) is converted to rectilinear motion.

The distal end portion of the drive rack axis 83 is fixed integrally to a first nozzle fixing surface 43a constituting the nozzle block 43. Accordingly, as the drive rack axis 83 advances or retreats, each of the tube path cleaning nozzles 31, 32, and 33 as well as the nozzle block 43 advance or retreat in the axial direction for insertion or removal.

It should be noted that when the tube path cleaning nozzles 31, 32, and 33 are inserted into the opening portions 25h of the coupling members 25d and 25e and into the opening portion 25g of the coupling members 25f, a control portion (not shown) of the cleaning/disinfecting apparatus 2C outputs a control signal instructing the removable drive motor 81 to rotate, for example, clockwise.

On the other hand, when the tube path cleaning nozzles 31, 32, and 33 are removed from the opening portions 25h of the coupling members 25d and 25e and from the opening portion 25g of the coupling members 25f, the control portion outputs a control signal instructing the removable drive motor 81 to rotate counterclockwise.

Then, the control portion calculates each distance on the basis of reflected signal from the ultrasonic sensor. When the calculated distance reaches a predetermined insertion complete distance or a cleaning/disinfecting wait position, the control portion outputs a rotation stop signal to the removable drive motor 81.

Accordingly, the same effect and advantages as described above can be obtained. It should be noted that the other configuration is the same as that of the aforementioned embodiment, and thus the same symbols are assigned to the same members and their description is omitted.

In addition, the present invention is not limited to the aforementioned embodiments, but various modifications can be made without departing from the scope and spirit of the present invention.

## Claims

1. An medical instrument cleaning/disinfecting system comprising:
a medical instrument provided with a tube path therein and whose external surface has an opening portion communicatively connected to the tube path;
a connection member provided with a connection portion capable of being connected to the medical instrument and has a communicative connection path communicatively connected to the tube path through the opening portion when the connection portion is connected;
a first detection portion for detecting that the medical instrument is placed in a predetermined position of a bath portion with a predetermined depth enough to place the medical instrument;
an operation portion for moving the connection member toward the medical instrument on the basis of the detection by the first detection portion;
a second detection portion for detecting a connection between the connection member and the medical instrument; and
a control portion for supplying a fluid from a fluid supply source to the communicative connection path on the basis of the detection by the second detection portion.

2. The medical instrument cleaning/disinfecting system according to claim 1, wherein the bath portion has a positioning portion for placing the medical instrument in a predetermined position, and the operation portion moves the connection member toward the medical instrument positioned by the positioning portion.

3. The medical instrument cleaning/disinfecting system according to claim 2, wherein the operation portion has a biasing portion for biasing the connection member toward the medical instrument.

4. The medical instrument cleaning/disinfecting system according to claim 3, wherein the biasing portion is an elastic member.

5. The medical instrument cleaning/disinfecting system according to claim 1 or 4, wherein the operation portion has a holding portion capable of holding the elastic member in an elastically deformed state.

6. The medical instrument cleaning/disinfecting system according to claim 5, wherein the holding portion is a latch solenoid.

7. The medical instrument cleaning/disinfecting system according to claim 2, wherein the operation portion is an advance/retreat portion for advancing and retreating the connection member to and from the medical instrument.

8. The medical instrument cleaning/disinfecting system according to claim 7, wherein the advance/retreat portion is provided with a drive portion for generating a predetermined drive power and a transmission portion for transmitting the drive power of the drive portion to the connection member.

9. The medical instrument cleaning/disinfecting system according to claim 8, wherein the drive portion is an electric motor.

10. The medical instrument cleaning/disinfecting system according to claim 1, wherein a plurality of tube paths are configured to have a plurality of communicative connection paths corresponding to each of the tube paths.

11. The medical instrument cleaning/disinfecting system according to claim 10, wherein when at least one of the plurality of tube paths is an aspiration tube path capable of being connected to an aspiration portion, an aspiration tube path opening portion serving as an opening portion communicatively connected to the aspiration tube path is spaced by a predetermined distance from other opening portions.
